Europäisches Patentamt

European Patent Office

Office européen des brevets

(19)

(11) Publication number:
**0 326 013**
**A2**

## EUROPEAN PATENT APPLICATION

(12)

(21) Application number: **89100851.8**

(22) Date of filing: **19.01.89**

(51) Int. Cl.4: **C07K 13/00 , C12P 21/02 ,**
**C12N 15/00**

The applicant has filed a statement in accordance with Rule 28 (4) EPC (issue of a sample only to an expert). Accession number(s) of the deposit(s): FERM BP-1350.

Claims for the following Contracting States: ES + GR.

(30) Priority: **28.01.88 JP 15762/88**

(43) Date of publication of application:
**02.08.89 Bulletin 89/31**

(84) Designated Contracting States:
**AT BE CH DE ES FR GB GR IT LI LU NL SE**

(71) Applicant: **HOECHST JAPAN LIMITED**
**10-16, 8-chome, Akasaka, Minato-ku**
**Tokyo(JP)**

(72) Inventor: **Toba, Mari**
**1-37-23, Kishimachi**
**Kawagoe-shi Saitama-ken(JP)**
Inventor: **Tone, Masahide**
**Forble Sekine A-201 345-2 Kitairiso**
**Sayama-shi Saitama-ken(JP)**
Inventor: **Kikuno, Reiko**
**Villa Charman 102 561-5 Kume**
**Tokorozawa-shi Saitama-ken(JP)**
Inventor: **Hashimoto, Tamotsu**
**1-5-10-309, Sakae-cho**
**Asaka-shi Saitama-ken(JP)**

(74) Representative: **Klein, Otto, Dr. et al**
**Hoechst AG Zentrale Patentabteilung**
**Postfach 80 03 20**
**D-6230 Frankfurt am Main 80(DE)**

(54) Novel proteins derived from human alpha2-plasmin inhibitor or proteins similar thereto.

(57) An $\alpha_2$-plasmin inhibitor ($\alpha_2$-PI) derivative which has essentially the amino acid sequence of $\alpha_2$-PI but for the reactive site cleavable with plasmin, which reactive site is either deleted or replaced by the reactive site of an antithrombin active serine protease inhibitor cleavable with thrombin, prevents blood coagulation and is useful in the therapy of cardiovascular disturbances or diseases.

EP 0 326 013 A2

## Novel proteins derived from human $\alpha_2$-plasmin inhibitor or proteins similar thereto

Background of the Invention

(1) Field of the Invention

The present invention provides novel proteins derived from human plasma $\alpha_2$-plasmin inhibitor (called $\alpha_2$-PI hereinbelow) or proteins similar thereto and a process for preparing those by virtue of the genetic engineering techniques. The protein possesses a blood coagulation-preventing activity and can be used for the preventive treatment or therapy of cardiovascular disturbances or diseases.

(2) Description of the Prior Art

In recent years attention has been drawn to tissue plasminogen-activating factor, human protein C and similar substances as new thrombolytic or blood coagulation-preventing agents in place of conventional thrombolytic agents such as urokinase and antithrombin III (called AT-III hereinbelow). $\alpha_2$-PI, one of the serine protease inhibitors occurring in blood, is a substance which accelerates blood coagulation and does not prevent blood coagulation. Surprisingly, we have found that by modifying part of the peptide of $\alpha_2$-PI a protein is obtained which is active in inhibiting blood coagulation. The object of the invention, therefore, is to provide new proteins having a blood coagulation-preventing activity.

Previously we filed a patent application relating to $\alpha_2$-PI and DNA coding for the same as European Patent Publication A1 0257630. $\alpha_2$-PI is a protein composed of 452 amino acids the amino terminal of which is Asn. Gln. Glu. Gln. Val. Ser. Pro. Leu. Thr. Leu. and the carboxyl terminal of which is Leu. Lys. Leu. Val. Pro. Pro. Met. Glu. Glu. Asp. Tyr. Pro. Gln. Phe. Gly. Ser. Pro. Lys. The protein is cleaved with plasmin between arginine at the 364th position and methionine at the 365th position from the N-terminal to exert a serine protease-inhibitor activity which accelerates blood coagulation.

$\alpha_2$-PI is also known to bind to fibrin via the above-mentioned peptide at the amino terminal by hydrogen bonding in the purified system or by covalent bonding with factor XIII in plasma (Aoki, N. et al. Thromb. Res., 19; 149-155, 1980). It is also known that $\alpha_2$-PI has an affinity with plasminogen by hydrogen bonding to the above-mentioned peptide at the carboxyl terminal (Moroi, M. et al. J. Biol. Chem., 251; 5956-5965, 1976). These properties are considered to be essential for the high substrate specificity of $\alpha_2$-PI with plasmin.

Therefore, we have conceived a derivative modified so as to lose the serine protease inhibitor activity while maintaining the amino acid sequences of the amino terminal and of the carboxyl terminal which derivative antagonistically inhibits the serine protease inhibitor activity of $\alpha_2$-PI.

Detailed Description of the Invention

The novel proteins of the invention are characterized by having the reactive site cleavable with plasmin and its flanking region of human plasma $\alpha_2$-plasmin inhibitor or a protein similar thereto (a) deleted or (b) replaced by the reactive site cleavable with thrombin or its flanking region of an antithrombin active protein and containing an amino terminal of Asn. Gln. Glu. Gln. Val. Ser. Pro. Leu. Thr. Leu. and a carboxyl terminal of Leu. Lys. Leu. Val. Pro. Pro. Met. Glu. Glu. Asp. Tyr. Pro. Gln. Phe. Gly. Ser. Pro. Lys.

$\alpha_2$-PI as specified in the invention designates a compound of the amino acid sequence described in European Patent Publication A1 0257630. The amino acid sequence is shown in Table 1. A "protein similar to $\alpha_2$-PI" means protein that is identical or, at least, similar on the whole and equivalent in effect to $\alpha_2$-PI though being different from $\alpha_2$-PI in substituting one or more of the amino acids, adding one or more amino acids, deleting one or more amino acid sequence, or a combination of these substitutions, additions and deletions.

The reactive site cleavable with plasmin and its flanking region of $\alpha_2$-PI designates the amino acid sequence of from 2 to approximately 60 amino acids involving arginine at the 364th position and methionine at the 365th position and its flanking region of $\alpha_2$-antiplasmin. The number of the amino acids involved therein may appropriately be changed. Particularly preferable is a region composed of 16 amino acid residues from alanine at the 357th position through valine at the 372nd position, a region composed of 44 amino acid residues from serine at the 348th position through valine at the 391st position or a region composed of 58 amino acid residues from serine at the 348th position through leucine at the 405th position.

Representative of the antithrombin active serine protease inhibitor is AT-III. A protein made by modifying a serine protease inhibitor other than AT-III so as to be cleaved with thrombin may be handled in the same way as above. AT-III is a substance that inhibits the activity of serine protease such as thrombin and is opposite to $\alpha_2$-PI by its blood coagulation inhibitory activity. The amino acid sequence of AT-III is described in Proc. Natl. Acad. Sci. USA, pp. 1845-1848 (1983). It is known that inhibition of thrombin by AT-III is caused by the cleavage of AT-III with a serine protease, thrombin, between arginine at the 393rd position and serine at the 394th position from the amino terminal of AT-III to form a covalent bond between the serine residue of the active site of thrombin and the arginine residue of the reactive site of AT-III (Harpel, P.C. et al. (1976) Prog. Hemostasis Tromb. vol. 3, pp. 145-189).

The reactive site of AT-III cleavable with thrombin and its flanking region designates an amino acid sequence of from 2 to about 60 amino acids including arginine at the 393rd position and serine at the 394th position and flanking region thereof. Particularly preferable is a region composed of 20 amino acid residues from serine at the 385th position through alanine at the 404th position or a region composed of 48 amino acid residues from asparagine at the 376th position through methionine at the 423rd position. The amino acids in these regions may be changed by substitution with or addition of other amino acids or deletion within such an extent as producing no change of the activity. In substituting the reactive site and its flanking region of $\alpha_2$-PI with the reactive site and its flanking region of AT-III, it is preferable either to substitute the 16 amino acid residues of 357th to 372nd of $\alpha_2$-PI with the 20 amino acid residues of 385th to 404th of AT-III or to substitute the 44 amino acid residues of 348th to 391st of $\alpha_2$-PI with the 48 amino acid residues of 376th to 423rd of AT-III. The region of $\alpha_2$-PI to be substituted and the region of AT-III to substitute can be determined on the basis of alignment of the amino acid sequences in the reactive site and its flanking region. As a matter of fact, substitution can be accomplished with a region corresponding in alignment to the region to be substituted. The amino acid sequence from asparagine at 376th position through methionine at 423th of AT-III is shown in Table 2.

It is known that whereas native $\alpha_1$-antitrypsin (called $\alpha_1$-AT hereinbelow) does not have antithrombin activity, a modified one having substitution of methionine at 358th position from the N-terminal with arginine has antithrombin activity (Nature, vol. 313, pp. 149-151 (1985)). The substitution may also be effected with the reactive site and its flanking region of such artificial antithrombin active serine protease inhibitor. It is also desirable as in the case with AT-III to determine the region of $\alpha_2$-PI to be substituted and the region of an antithrombin active substance to substitute on the basis of alignment of the two.

The hybrid protein of the invention is prepared by virtue of genetic engineering techniques. Therefore, the present invention relates also to DNAs coding for said proteins and a process for preparing said hybrid proteins by means of genetic engineering using said DNA.

Nucleotide sequence and other characteristics of the gene coding for $\alpha_2$-PI are described in European Patent Publication A1 0257630. The plasmid p$\alpha$AP3 containing the gene (APHL) has been transferred into E. coli cell and is deposited as FERM BP-1350 at the Institute of Microbiological Industrial Technology of the Agency of Industrial Science and Technology. It is therefore convenient to excise the gene coding for $\alpha_2$-PI from p$\alpha$AP3. The gene thus obtained may be modified for nucleotides within such an extent that amino acids to be encoded are not changed. Change of nucleotides to result in change of amino acids may also be made within such an extent that there is no change in activity.

It is convenient to connect shorter DNA fragments chemically synthesized in accordance with the amino acid sequence and the nucleotide sequence of a gene published in literatures for the preparation of a gene corresponding to the amino acid sequence of the reactive site and its flanking region of an inhibitor such as AT-III. It is also feasible to isolate a DNA portion as needed by cleavage of the gene coding for AT-III. Modification of the gene coding for $\alpha_2$-PI is accomplished by deleting the portion to be missing, in that the gene for $\alpha_2$-PI is treated with a restriction enzyme or by incorporating a DNA fragment corresponding to the amino acid sequence of the reactive site and its flanking region of AT-III into this deletion site containing the missing region.

To the gene thus obtained are added functions necessary for expression such as a promoter and a terminator corresponding to a suitable host in order to prepare an expression plasmid.

The expression vector employing animal cells as host contains, in addition to a gene coding for the protein of the invention, a promoter of an eucaryotic organism to lead initiation of the transcription, a polyadenylation site to facilitate termination of the transcription and a gene coding for a selection marker necessary for preparing the plasmid DNA using E. coli. As examples of the promoter of an eucaryotic organism are mentioned SV40 early promoter, the promoter of mouse mammary tumor virus (called MMTV for short) and the promoter of Drosophila heat shock protein 70 (called HSP 70 for short). As an example of the transcription-termination signal is mentioned the polyadenylation site of SV40 early mRNA.

The expression vector employing a procaryotic organism as host contains, in addition to a gene coding for the protein of the invention, a promoter of a procaryotic organism to instruct initiation of the transcription, a terminator of a procaryotic organism to instruct termination of the transcription and a gene coding for an appropriate selection marker. As an example of the host procaryotic cells is mentioned Escherichia coli - (called E. coli for short). As examples of the promoter are mentioned trp promoter or tac promoter of E. coli which is efficient in transcription initiation. As an example of the terminator is mentioned rrnB ribosomal RNA terminator which is a strong termination signal. The gene coding for $\alpha_2$-PI which is contained in p$\alpha$AP3 deposited as FERM BP-1350 is shown in Table 1.

The DNA sequence from 1 to 6 is the EcoRI site originating from the EcoRI linker used in the cloning and the DNA sequence from 7 to 24 is a non-coding region upstream of the 5'-end. The DNA sequence of the nucleotides from 25 to 141 is corresponding to the peptide contained only in the precursor of $\alpha_2$-PI (so-called signal peptide coding portion or anchor portion). The DNA sequence from 142 to 1497 is the subject portion coding for $\alpha_2$-PI. The TGA triplet from 1498 to 1500 is a stop codon. There are in the DNA sequence from 1501 ff. a non-coding region downstream of the 3'-end, a poly A signal and EcoRI site originating from the EcoRI linker used in the cloning, all of which are omitted in Table 1.

Where animal cells are used as host, the gene carrying the anchor portion of the nucleotide positions from 25 to 141 is incorporated into the expression vector. Where procaryotic cells are used as host, a gene in which the anchor portion is deleted and, instead, the translation-initiating codon ATG is added is incorporated into the expression vector.

In the DNA sequence shown in Table 1, there are SacI sites cleaved at the positions 1181 and 1355. Therefore, it is convenient to use the SacI sites so that the reactive site and its flanking region of $\alpha_2$-PI can be easily deleted or substituted.

The present invention, furthermore, is concerned with plasmids containing DNA arranged to express the desired protein using the above-described DNA and also the procaryotic or eucaryotic cells transformed with the plasmid DNA.

The invention will be described in details with reference to examples.

Example 1

Preparation of an expression plasmid in E. coli using $\alpha_2$-plasmin inhibitor cDNA

The plasmid p$\alpha$AP3 obtained by culturing E. coli K12/Om214 (FERM BP-1350) was cleaved with EcoRI and blunt-ended with T4 DNA polymerase followed by cleavage with HindIII at the nucleotide position of 1427 in Table 1. The amino terminal-containing fragment was inserted into pUC18 (ampicillin resistant, available from the gene bank of the Japanese National Institute of Health, Deposit No. VE 007) between HindIII site blunt-ended with T4 DNA polymerase and PstI site to give p$\alpha$AP5. Separately, a fragment containing the region coding for the carboxyl terminal of $\alpha_2$-PI obtained by cleavage with FokI at the nucleotide position of 1507, subsequent polishing the ends with T4 DNA polymerase and then cleavage with HindIII at the nucleotide position of 1427 was inserted between the HincII site and the HindIII site of pHSG 399 (chloramphenical resistant, available from the same gene bank as above, Deposit No. VE 024) to give p$\alpha$AP6. Then, p$\alpha$AP5 and p$\alpha$AP6, both after cleavage with HindIII, were ligated, and a plasmid which was double resistant to ampicillin and chloramphenicol was selected to give p$\alpha$AP7. The p$\alpha$AP7 contained the entire region encoding $\alpha_2$-PI (1476 bp of the DNA sequence from 25 to 1500), and a fragment containing the entire region was excised by cleavage at AccI site (in 5' upstream region) and at XbaI site (in 3' downstream region).

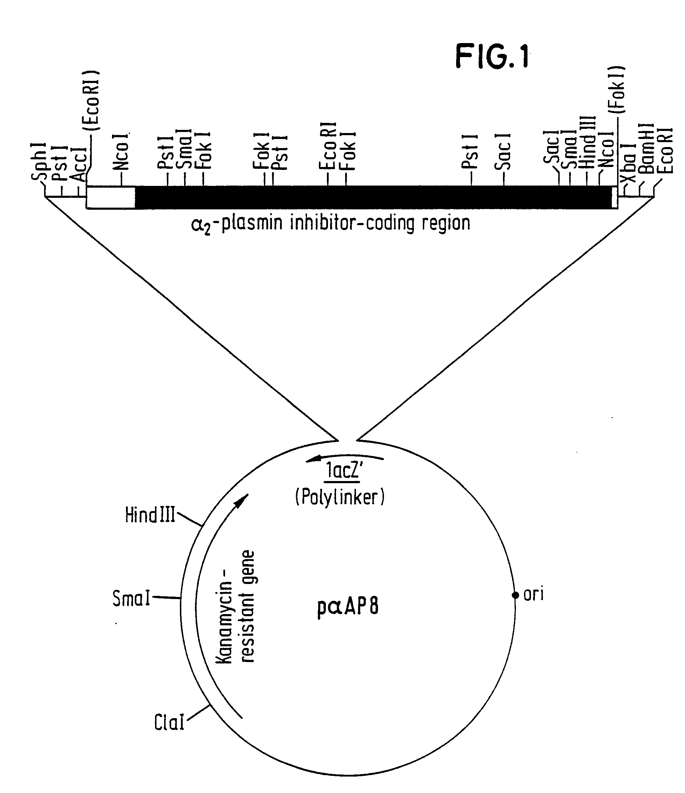

Separately, a cloning vector called pHSG297 was prepared in which the nucleotide sequence between the SacI site and the SmaI site of pHSG298 (kanamycin resistant, available from the same gene bank as above, Deposit No. VE 018) was deleted by cleaving with the enzymes and polishing the ends with T4 DNA polymerase. Between the AccI site and the XbaI site of the cloning vector was inserted DNA for $\alpha_2$-PI between the AccI site and the XbaI site of p$\alpha$AP7 to give p$\alpha$AP8 (Fig. 1).

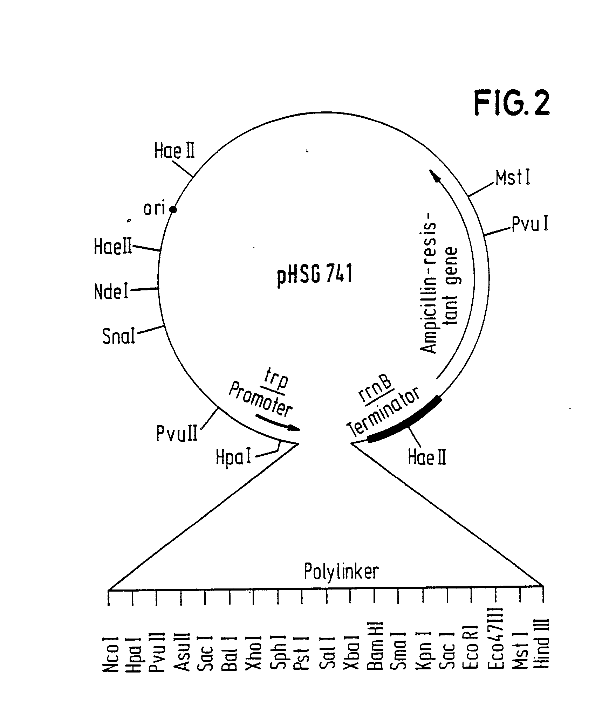

On the other hand, pHSG741 (Fig. 2, ampicillin resistant, available from the same bank as above, Deposit No. VE 040) was an expression vector containing a tryptophan promoter and rrnB terminator for a ribosomal RNA gene. Between these two regions there were the cloning sites NcoI, PstI, EcoRI and HindIII arranged in this order. First, between the NcoI site and the PstI site of the plasmid was inserted the following fragment which was synthesized chemically:

5′ CATGAACCAGGAGCAGGTGTCCCCACTTACCCTCCTCAAGTTGGGCAACC
3′ TTGGTCCTCGTCCACAGGGGTGAATGGGAGGAGTTCAACCCGTTGG


AGGAGCCTGGTGGCCAGACTGCCCTGAAGAGTCCCCCAGGAGTCTGCA 3′
TCCTCGGACCACCGGTCTGACGGGACTTCTCAGGGGGTCCTCAG 5′


The cohesive end CATG appearing at the beginning of the fragment was complementary to the cohesive end of the NcoI site appearing immediately after the tryptophan promoter and the SD sequence of pHSG741, and the ATG functioned as the translation-initiating codon for $\alpha_2$-PI. The DNA sequence from 142 ff. corresponded to the second codon and the following portion except for the leader sequence of said protein. They coded for Asn-Gln-Glu-Gln ff. after the first amino acid. The cohesive end at the end of the fragment was complementary to the cohesive end of the PstI site at the nucleotide position of 236.

Next, between the PstI site and the EcoRI site of the plasmid the DNA portion was inserted for $\alpha_2$-PI obtained from p$\alpha$AP9 which was a fragment between PstI site at the nucleotide position of 236 and the EcoRI site at the nucleotide position of 676.

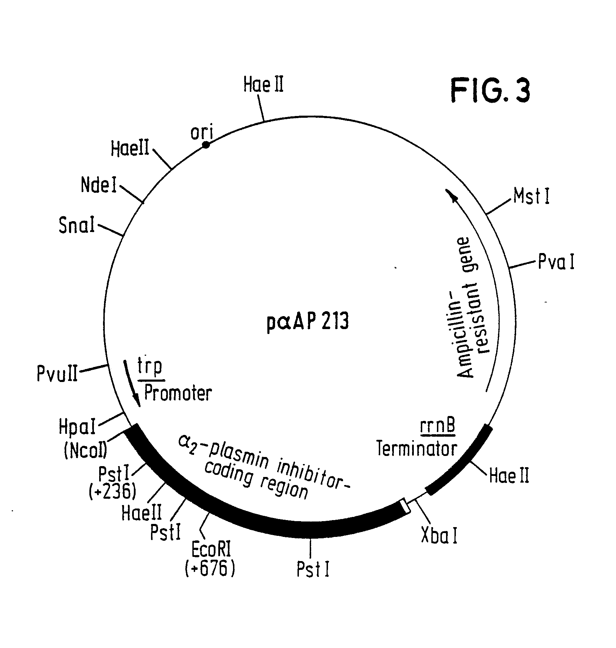

Next, between the EcoRI site and the blunt-ended HindIII site of the plasmid was inserted the DNA fragment obtained from p$\alpha$AP8 which was from the EcoRI site at the nucleotide position of 676 through blunt-ended XbaI site in the 3′ downstream region. The process resulted in regeneration of XbaI sites and thus the construction of p$\alpha$AP213 was completed (Fig. 3).


## Example 2


Preparation of plasmid DNA capable of expressing in E. coli proteins in which 58 amino acids of the reactive site and its flanking region of $\alpha_2$-PI are deleted

p$\alpha$AP213 was digested with SacI to cause cleavages at the nucleotide positions of 1181 and 1355 to remove the nucleotide sequence corresponding to 58 amino acids from the 348th to 405th base of $\alpha_2$-PI. The product was ligated to produce the plasmid p$\alpha$AP216 coding for the protein in which 58 amino acids from positions 348 to 405 are deleted. The protein encoded by p$\alpha$AP216 was called PI-R-O.


## Example 3


Preparation of plasmid DNA capable of expressing in animal cells proteins in which the reactive site and its flanking region of $\alpha_2$-PI are deleted

In p$\alpha$AP8, one of the plasmides prepared in Example 1, both ends of the cDNA coding for $\alpha_2$-PI were put between AccI site and XbaI site. The portion of the p$\alpha$AP8 between two SacI sites coding for 58 amino acid residues covering the reactive site and its flanking region of $\alpha_2$-PI was removed by SacI digestion and re-ligation with T4 DNA ligase to prepare the plasmid p$\alpha$AP9.

Next, a plasmid consisting of a DNA fragment containing SV40 early promoter, SV40 early mRNA polyadenylation site and DNA replication-initiating site in animal cells (which was isolated from pKSV-10 commercially available from Pharmacia Japan or SV40 DNA commercially available from Bethesda Research Laboratories, USA) and a DNA replication-initiating site and an ampicillin-resistance gene of pBR 322 (commercially available from Takara Shuzo and others) was prepared. Between the SV40 early promoter and the SV40 early mRNA polyadenylation site of the plasmid was incorporated a chemically synthesized polylinker DNA fragment containing the AccI site and XbaI site. Between the AccI site and the XbaI site of the polylinker portion a DNA fragment from p$\alpha$AP9 was incorporated containing DNA for $\alpha_2$-PI without the reactive site and its flanking region which was excised by AccI and XbaI digestions to complete the expression plasmid in animal cells.

Example 4

Preparation of plasmid capable of expressing in E. coli proteins in which 44 amino acid residues from positions 348 to 391 containing the reactive site of $\alpha_2$-PI are substituted with 48 amino acid residues from 376th to 423rd of AT-III

pαAP213 was digested with SacI to cause cleavages at the nucleotide positions of 1181 and 1355 thereby removing the nucleotide sequence corresponding to 58 amino acids from positions 348 to 405 of $\alpha_2$-PI. The DNA fragment coding for the portion from asparagine at the position 376 through methionine at the position 423 of AT-III and the portion from glycine at the position 392 through leucine at the position 405 of $\alpha_2$-PI was synthesized chemically in two fragments as follows:

Fragment A

```
5'   CAATGAGGAAGGCAGTGAGGCGGCGGCCAGTACGGCTGTTGTGATTGCTGGCCGAAGCT
3'   TCGAGTTACTCCTTCCGTCACTCCGCCGCCGGTCATGCCGACAACACTAACGACCGGCTTCGA


     TAAACCCCAACAGGGTGACTTTCAAGGCCAACC        3'
     ATTTGGGGTTGTCCCACTGAAAGTTCCGGTTGGCCGG        5'
```

Fragment B

```
5'   GGCCGTTCCTCGTTTTCATCAGAGAGGTTCCTCTGAACACTATTATCTTCATGGGATC
3'       CAAGGAGCAAAAGTAGTCTCTCCAAGGAGACTTGTGATAATAGAAGTACCCTAG


     CGTGAGGAACCCCAACCCCAGTGCACCGCGGGAGCT        3'
     GCACTCCTTGGGGTTGGGGTCACGTGGCGCCC        5'
```

The fragment A and the first half of the fragment B were the portion corresponding to the amino acid residues from positions 376 to 423 of AT-III, and the latter half of the fragment B was the portion corresponding to the amino acid residues from positions 392 to 405 of $\alpha_2$-PI. The sequence of cDNA for AT-III in the fragments A and B was in accordance with Proc. Natl. Acad. Sci. USA, vol. 80, pp. 1845-1848 (1983). It was to be noted that the restriction sites for BglI, HindIII, XmaIII and BamHI were introduced into the fragments arranged in this order from the upstream of the coding region in such a way that the amino acid sequence was not changed. Then, the fragment A and the fragment B were connected to make the contiguous fragment A + B. The fragment A + B was inserted between the two SacI sites of pαAP213, and a clone with BglI, HindIII, XmaIII and BamHI sites arranged in this order from the upstream was selected to give pαAP214. The protein encoded by pαAP214 was called PI-RAT-L.

Example 5

Preparation of a plasmid capable of expressing in E. coli proteins in which 16 amino acid residues from positions 357 to 372 containing the reactive site of $\alpha_2$-PI is substituted with 20 amino acid residues from positions 385 to 404 of AT-III

pαAP213 was digested with SacI to cause cleavages at the nucleotide positions of 1181 and 1355 thereby removing the nucleotides corresponding to 58 amino acids from positions 348 to 405 of $\alpha_2$-PI. The DNA fragment coding for a peptide corresponding to the amino acids from positions 348 to 356 of $\alpha_2$-PI, the amino acids from positions 385 to 404 of AT-III and the amino acids from positions 373 to 405 of $\alpha_2$-PI was synthesized chemically in two fragments as follows:

Fragment C

```
5'        CAGCGAGGTCGGCGTGGAGGCGGCGGCCAGTACGGCTGTTGTGATTGCTGGCCGAAGCT

3'   TCGAGTCGCTCCAGCCGCACCTCCGCCGCCGGTCATGCCGACAACACTAACGACCGGCTTCGA


       TAAACCCCAACAGGGTGACTTTCAAGGCCAACC      3'

       ATTTGGGGTTGTCCCACTGAAAGTTCCGGTTGGCCGG      5'
```

Fragment D

```
5'     GGCCGTTCCTCTTCTTCATCTTCGAGGACACCACAGGCCTTCCCCTCTTCGTGGGATC

       CAAGGAGAAGAAGTAGAAGCTCCTGTGGTGTCCGGAAGGGGAGAAGCACCCTAG


       CGTGAGGAACCCCAACCCCAGTGCACCGCGGGAGCT      3'

       GCACTCCTTGGGGTTGGGGTCACGTGGCGCCC      5'
```

The fragments were ligated to form the fragment C + D. The fragment C + D was inserted between the two SacI sites of p$\alpha$AP213, and the plasmid thus obtained was named p$\alpha$AP215. The protein encoded by p$\alpha$AP215 was called PI-RAT-S.

Example 6

Expression in E. coli K-12 of $\alpha_2$-PI encoded by p$\alpha$AP213 and PI-R-O encoded by p$\alpha$AP216 in which the reactive site of $\alpha_2$-PI is deleted, and antiplasmin activities thereof

E. coli K-12 W 3110 (available from ATCC, ATCC 27325) were transformed with p$\alpha$AP213, p$\alpha$AP216 or pHSG741 and ampicillin-resistant microorganisms were selected. The microorganisms were cultivated in LS medium (Hashimoto-Gotoh, T. and Inselburg, J., described in J. Bacteriol., 139 (1979), 608-619) in the presence of ampicillin at 37°C overnight. 100 ml of LS medium was innoculated with 1 ml of the culture. After being cultivated with shaking at 37°C for 1 hr. and 45 min., the cells were recovered by centrifugation and suspended in casamino acid medium (10.5 g, $K_2HPO_4$; 4.5 g, $KH_2PO_4$; 2.0 g, $(NH_4)SO_4$; 2.0 g, $NH_4Cl$; 0.5 g, Na-citrate $2H_2O$; 0.5 ml, 1% thiamine hydrochloride; 1% glucose; 1% casamino acid for vitamin assay; 100 $\mu$g/ml, ampicillin; 1 l. water). Starting at this point as 0 hr. cultivation with shaking was continued and at each point of time after 1.5 hr., 3.5 hr. and 5.0 hr. cultivation the liquid culture in a volume corresponding to $OD_{550} = 0.05$ (7.5 $\mu$l to 45 $\mu$l) was collected. The cells were recovered and suspended in a buffer solution for SDS-PAGE followed by electrophoresis in a 10% acrylamide gel and Western blot analysis. The primary antibody used was rabbit anti-human $\alpha_2$-PI antisera (CALBIOCHEM® 178222). Both with p$\alpha$AP213 and p$\alpha$AP216, no band was observed at 0 hr. but distinct bands were observed at each point of time after 1.5 hr., 3.5 hr. and 5 hr. cultivation, respectively corresponding to respective molecular weights of about 52 Kd and about 40 Kd. Using E. coli W3110 having pHSG741 as a control no band was observed at any point of time. The lysates originated from p$\alpha$AP213, p$\alpha$AP216 and pHSG741 were analyzed by ELISA method using rabbit anti-human $\alpha_2$-plasmin inhibitor polyclonal antibody. As calculated in comparison with the standard curve using purified α₂-PI originated from human plasma there was observed immunoreactive α₂-PI in an amount of 28 μg/ml in the lysate (or 9.6 μg/ml in the liquid culture) for pαAP213 and 1.4 μg/ml in the lysate (0.48 μg/ml in the liquid culture) for pαAP216. It was to be noted that since the reactive site was missed in the latter there was high possibility of underestimation. In this analysis the experiment with pHSG741 gave the value below detectable limit ($\leq$ 0.2 μg/ml of the liquid culture).

40 ml of the liquid culture after 3.5 hr. cultivation was collected and centrifuged. The cells were suspended in 11 ml ($OD_{550} = 20$) of the buffer solution attached to the kit for measurement of α₂-PI manufactured by Sankyo Co., Ltd. [α₂-PI Color Test "Sankyo" (No. 48783)]. To 2.5 ml of the suspension were added 60 μl of 0.5 M EDTA, 300 μl of 5 mg/ml lysozyme and 140 μl of the above-mentioned buffer solution to make a total volume of 3 ml. The mixture was allowed to stand at 0°C for 30 min. followed by seven times treatment of sonication each for 10 sec. to crush the cells. The crushed cells were centrifuged at 11800 x g, and the liquid lysate was recovered. Total protein concentration including the lysozyme was 3.1 mg/ml. Antiplasmin activity in the liquid lysate derived from pαAP213 relative to that of human plasma were 80%, 140% and 220% per 125 μg, 250 μg and 500 μg protein, respectively. In the liquid lysate of E. coli cells having pαAP216, the value was about 10-20% and there was no significant difference from that of E. coli cells carrying pHSG741 used as a control.

Example 7

Expression if E. coli K-12 of α₂-PI encoded by pαAP213, PI-RAT-L encoded by pαAP214 and PI-RAT-S encoded by pαAP215, and antiplasmin activities thereof

E. coli K-12 W3110 (available from ATCC, ATCC27325) were transformed with pαAP213, pαAP214, pαAP215 and pHSG741, respectively, and ampicillin-resistant transformants were selected. These transformants were cultivated by the same procedures as in Example 6 and the cultures were analyzed by Western blotting. In all of the analyses with pαAP213, pαAP214 and pαAP215 no band was detected at 0 hr. but distinct bands corresponding to a molecular weight of about 52 Kd were detected at each point of time after 1.5, 3.5 and 5.0 hr. cultivation, respectively.

Using E. coli W3110 having pHSG741 as a control, there was observed no band at any point of time. Measurement was made for the lysate that originated from pαAP213 by ELISA method using rabbit anti-human α₂-plasmin inhibitor polyclonal antibody. There was observed 28 μg/ml of immunoreactive α₂-PI in the lysate (or 9.6 μg/ml in the liquid culture). It was 7-10 μg/ml for pαAP214 or pαAP215. As the latter two plasmids code for hybrid proteins with AT-III, it was highly possible that the values were underestimated. In the experiment with pHSG741 the value was below detectable limit ($\leq$ 0.2 μg/ml of the liquid culture).

40 ml of the liquid culture after 3.5 hr. cultivation was collected and centrifuged. The cells were suspended in 11 ml ($OD_{550} = 20$) of the buffer solution attached to the kit for measurement of α₂-PI manufactured by Sankyo Co., Ltd. [α₂ PI Color Test "Sankyo" (No. 48783)]. To 2.5 ml of the suspension were added 60 μl of 0.5 M EDTA, 300 μl of 5 mg/ml lysozyme and 140 μl of the above-mentioned buffer solution to make a total volume of 3 ml. The mixture was allowed to stand at 0°C for 30 min. followed by seven times treatment of sonication each for 10 sec. to crush the cells. The crushed cells were centrifuged at 11800 x g, and the liquid lysate was recovered. Total protein concentration including the lysozyme was 3.1 mg/ml. Antiplasmin activity in the liquid lysate derived from pαAP213 relative to that of human plasma were 80%, 140% and 220% per 125 μg, 250 μg and 500 μg protein, respectively. The activities in the lysates of E. coli cells carrying pHSG741 as a control, pαAP214 and pαAP215 were in the range of 10% to 20%. The above results indicated that α₂-PI was produced in E. coli with pαAP213, and the hybrid proteins with AT-III were produced in E. coli with pαAP214 and pαAP215, and the protein produced with pαAP213 was reactive with anti-α₂-PI antiserum and had an antiplasmin activity, and the proteins produced with pαAP214 or pαAP215 were reactive with anti-α₂-plasmin inhibitor antiserum but had no antiplasmin activity.

Example 8

Purification by HPLC of α₂-PI and variant defective in the reactive site thereof

Lysates were prepared as described in Example 5 of E. coli with pαAP213, pαAP216 and pHSG741, respectively. The lysate was filtered through MILLEX filter (manufactured by Nippon Millipore, SLV025LS) and then subjected to gel filtration by HPLC as described below to partially purify $\alpha_2$-PI and the variant defective in the reactive site thereof. The column used was TSK-G3000 SWG (21.5 mm x 600 mm) (Toso K.K.) through which column was passed 10 mM sodium phosphate buffer solution containing 0.75 M NaCl (pH 7.2) at a flow rate of 3 ml/min. in a total volume of about 210 ml. To the HPLC was applied 1 ml of the above-mentioned lysate, which was collected in 3 ml fractions. It was confirmed by SDS-10% acrylamide gel electrophoresis that $\alpha_2$-PI with a molecular weight of 52 Kd was eluted at 70 min. after starting the elution and the defective variant with a molecular weight of about 40 Kd at 90 min. after starting the elution. The $\alpha_2$-plasmin inhibitor (p$\alpha$AP213) and the variant defective in the reactive site thereof (p$\alpha$AP216) thus obtained were contained at concentrations of 114 $\mu$g and 5.7 $\mu$g per ml of the liquid colture of E. coli respectively, as normalized by purified human $\alpha_2$-PI when measured by ELISA method using the above-mentioned polyclonal antibody. The purity was about 15-fold higher than that of starting material, and total amounts of the protein were 1880 $\mu$g and 2080 $\mu$g, respectively. SDS-PAGE and Western blot analysis, however, showed a distinct band of approximately equal width which was the same as in the experiment using the lysate before purification. These results suggested that apparent difference in the amount of protein as determined by ELISA method did not represent actual difference in amount but reflect difference in affinity to the antibody. (As difference in affinity of the $\alpha_2$-PI produced in E. coli to the antibody against $\alpha_2$-PI originated from human serum is uncertain, it is likely that the absolute value does not reflect mean value.)

## Table 1

```
*1
                                                         GAA. TTC. CGG. CTG. GCA. GGG. GAG


*22.
AAC. ATG. GCG. CTG. CTC. TGG. GGG. CTC. CTG. GTG. CTC. AGC. TGG. TCC. TGC. CTG. CAA. GGC. CCC. TGC


*82
TCC. GTG. TTC. TCC. CCT. GTG. AGC. GCC. ATG. GAG. CCC. TTG. GGC. TGG. CAG. CTA. ACT. AGC. GGG. CCG


*142
AAC. CAG. GAG. CAG. GTG. TCC. CCA. CTT. ACC. CTC. CTC. AAG. TTG. GGC. AAC. CAG. GAG. CCT. GGT. GGC
Asn-Gln-Glu-Gln-Val-Ser-Pro-Leu-Thr-Leu-Leu-Lys-Leu-Gly-Asn-Gln-Glu-Pro-Gly-Gly
                                                                                    20

*202                                                         *216
CAG. ACT. GCC. CTG. AAG. AGT. CCC. CCA. GGA. GTC. TGC. AGC. AGA. GAC. CCC. ACC. CCA. GAG. CAG. ACC
Gln-Thr-Ala-Leu-Lys-Ser-Pro-Pro-Gly-Val-Cys-Ser-Arg-Asp-Pro-Thr-Pro-Glu-Gln-Thr
                                                                                    40

*262
CAC. AGG. CTG. GCC. CGG. GCC. ATG. ATG. GCC. TTC. ACT. GCC. GAC. CTG. TTC. TCC. CTG. GTG. GCT. CAA
His-Arg-Leu-Ala-Arg-Ala-Met-Met-Ala-Phe-Thr-Ala-Asp-Leu-Phe-Ser-Leu-Val-Ala-Gln
                                                                                    60

*322
ACG. TCC. ACC. TGC. CCC. AAC. CTC. ATC. CTG. TCA. CCC. CTG. AGT. GTG. GCC. CTG. GCG. CTG. TCT. CAC
Thr-Ser-Thr-Cys-Pro-Asn-Leu-Ile-Leu-Ser-Pro-Leu-Ser-Val-Ala-Leu-Ala-Leu-Ser-His
                                                                                    80

*382
CTG. GCA. CTA. GGT. GCT. CAG. AAC. CAC. ACG. TTG. CAG. AGG. CTG. CAA. CAG. GTG. CTG. CAC. GCA. GGC
Leu-Ala-Leu-Gly-Ala-Gln-Asn-His-Thr-Leu-Gln-Arg-Leu-Gln-Gln-Val-Leu-His-Ala-Gly
                                                                                    100

*442
TCA. GGG. CCC. TGC. CTC. CCC. CAT. CTG. CTG. AGC. CGC. CTC. TGC. CAG. GAC. CTG. GGC. CCC. GGC. GCG
Ser-Gly-Pro-Cys-Leu-Pro-His-Leu-Leu-Ser-Arg-Leu-Cys-Gln-Asp-Leu-Gly-Pro-Gly-Ala
                                                                                    120

*502
TTC. CGA. CTG. GCT. GCC. AGG. ATG. TAC. CTG. CAG. AAA. GGA. TTT. CCC. ATC. AAA. GAA. GAT. TTC. CTG
Phe-Arg-Leu-Ala-Ala-Arg-Met-Tyr-Leu-Gln-Lys-Gly-Phe-Pro-Ile-Lys-Glu-Asp-Phe-Leu
                                                                                    140

*562
GAA. CAA. TCC. GAA. CAG. CTA. TTT. GGG. GCA. AAG. CCC. GTG. AGC. CTG. ACG. GGA. AAG. CAG. GAA. GAT
Glu-Gln-Ser-Glu-Gln-Leu-Phe-Gly-Ala-Lys-Pro-Val-Ser-Leu-Thr-Gly-Lys-Gln-Glu-Asp
                                                                                    160

*622                                                         *676
GAC. CTG. GCA. AAC. ATC. AAC. CAA. TGG. GTG. AAG. GAG. GCC. ACG. GAG. GGG. AAG. ATT. CAG. GAA. TTC
Asp-Leu-Ala-Asn-Ile-Asn-Gln-Trp-Val-Lys-Glu-Ala-Thr-Glu-Gly-Lys-Ile-Gln-Glu-Phe
                                                                                    180

*682
CTC. TCT. GGG. CTG. CCG. GAA. GAC. ACC. GTG. TTG. CTT. CTC. CTC. AAC. GCC. ATC. CAC. TTC. CAG. GGT
Leu-Ser-Gly-Leu-Pro-Glu-Asp-Thr-Val-Leu-Leu-Leu-Leu-Asn-Ala-Ile-His-Phe-Gln-Gly
                                                                                    200
```

Table 1 (Continued)

```
*742
TTC. TGG. AGG. AAC. AAG. TTT. GAC. CCG. AGC. CTT. ACC. CAG. AGA. GAC. TCC. TTC. CAC. CTG. GAC. GAG
Phe-Trp-Arg-Asn-Lys-Phe-Asp-Pro-Ser-Leu-Thr-Gln-Arg-Asp-Ser-Phe-His-Leu-Asp-Glu
*802                                                                             220
CAG. TTC. ACG. GTG. CCC. GTG. GAA. ATG. ATG. CAG. GCC. CGC. ACG. TAC. CCG. CTG. CGC. TGG. TTC. TTG
Gln-Phe-Thr-Val-Pro-Val-Glu-Met-Met-Gln-Ala-Arg-Thr-Tyr-Pro-Leu-Arg-Trp-Phe-Leu
*862                                                                             240
CTG. GAG. CAG. CCT. GAG. ATC. CAG. GTG. GCT. CAT. TTC. CCC. TTT. AAG. AAC. AAC. ATG. AGC. TTT. GTG
Leu-Glu-Gln-Pro-Glu-Ile-Gln-Val-Ala-His-Phe-Pro-Phe-Lys-Asn-Asn-Met-Ser-Phe-Val
*922                                                                             260
GTC. CTT. GTA. CCC. ACC. CAC. TTT. GAA. TGG. AAC. GTG. TCC. CAG. GTA. CTG. GCC. AAC. CTG. AGT. TGG
Val-Leu-Val-Pro-Thr-His-Phe-Glu-Trp-Asn-Val-Ser-Gln-Val-Leu-Ala-Asn-Leu-Ser-Trp
*984                                                                             280
GAC. ACC. CTG. CAC. CCA. CCT. CTG. GTG. TGG. GAG. AGG. CCC. ACC. AAG. GTC. CGG. CTG. CCT. AAG. CTG
Asp-Thr-Leu-His-Pro-Pro-Leu-Val-Trp-Glu-Arg-Pro-Thr-Lys-Val-Arg-Leu-Pro-Lys-Leu
*1042                                                                           300
TAT. CTG. AAA. CAC. CAA. ATG. GAC. CTG. GTG. GCC. ACC. CTC. AGC. CAG. CTG. GGC. CTG. CAG. GAG. TTG
Tyr-Leu-Lys-His-Gln-Met-Asp-Leu-Val-Ala-Thr-Leu-Ser-Gln-Leu-Gly-Leu-Gln-Glu-Leu
*1102                                                                           320
TTC. CAG. GCC. CCA. GAC. CTG. CGT. GGG. ATC. TCC. GAG. CAG. AGC. CTG. GTG. GTG. TCC. GGC. GTG. CAG
Phe-Gln-Ala-Pro-Asp-Leu-Arg-Gly-Ile-Ser-Glu-Gln-Ser-Leu-Val-Val-Ser-Gly-Val-Gln
*1164                       *1181                                               340
CAT. CAG. TCC. ACC. CTG. GAG. CTC. AGC. GAG. GTC. GGC. GTG. GAG. GCG. GCG. GCG. GCC. ACC. AGC. ATT
His-Gln-Ser-Thr-Leu-Glu-Leu-Ser-Glu-Val-Gly-Val-Glu-Ala-Ala-Ala-Ala-Thr-Ser-Ile
*1222                        348                                    357         360
GCC. ATG. TCC. CGC. ATG. TCC. CTG. TCC. TCC. TTC. AGC. GTG. AAC. CGC. CCC. TTC. CTC. TTC. TTC. ATC
Ala-Met-Ser-Arg-Met-Ser-Leu-Ser-Ser-Phe-Ser-Val-Asn-Arg-Pro-Phe-Leu-Phe-Phe-Ile
*1282        364  365                          372                              380
TTC. GAG. GAC. ACC. ACA. GGC. CTT. CCC. CTC. TTC. GTG. GGC. AGC. GTG. AGG. AAC. CCC. AAC. CCC. AGT
Phe-Glu-Asp-Thr-Thr-Gly-Leu-Pro-Leu-Phe-Val-Gly-Ser-Val-Arg-Asn-Pro-Asn-Pro-Ser
*1342        *1355                      391                                     400
GCA. CCG. CGG. GAG. CTC. AAG. GAA. CAG. CAG. GAT. TCC. CCG. GGC. AAC. AAG. GAC. TTC. CTC. CAG. AGC
Ala-Pro-Arg-Glu-Leu-Lys-Glu-Gln-Gln-Asp-Ser-Pro-Gly-Asn-Lys-Asp-Phe-Leu-Gln-Ser
*1402               405                 *1427                                   420
CTG. AAA. GGC. TTC. CCC. CGC. GGA. GAC. AAG. CTT. TTC. GGC. CCT. GAC. TTA. AAA. CTT. GTG. CCC. CCC
Leu-Lys-Gly-Phe-Pro-Arg-Gly-Asp-Lys-Leu-Phe-Gly-Pro-Asp-Leu-Lys-Leu-Val-Pro-Pro
*1462                                              *1507                        440
ATG. GAG. GAG. GAT. TAC. CCC. CAG. TTT. GGC. AGC. CCC. AAG. TGA. GGG. GCC. GTG. GCT. GTG. GCA. TCC
Met-Glu-Glu-Asp-Tyr-Pro-Gln-Phe-Gly-Ser-Pro-Lys-***
```

EP 0 326 013 A2

## Table 2

Asn-Glu-Glu-Gly-Ser-Glu-Ala-Ala-Ala-<u>Ser</u>
376                                        385

Thr-Ala-Val-Val-Ile-Ala-Gly-Arg-Ser-Leu

Asn-Pro-Asn-Arg-Val-Thr-Phe-Lys-<u>Ala</u>-Asn
404

Arg-Pro-Phe-Leu-Val-Phe-Ile-Arg-Glu-Val

Pro-Leu-Asn-Thr-Ile-Ile-Phe-<u>Met</u>
423

### Brief Description of the Drawing

Fig. 1 shows restriction map of the plasmid pαAP8. In the figure (Fok I) and (EcoRI) are the sites eliminated by ligation.

Fig. 2 shows restriction map of the plasmid pHSG741.

Fig. 3 shows restriction map of the plasmid pαAP213. In the figure (NcoI) is the site eliminated by ligation.

### Claims

1. An $\alpha_2$-plasmin inhibitor ($\alpha_2$-PI) derivative having essentially the amino acid sequence of $\alpha_2$-PI but for the reactive site cleavable with plasmin, which reactive site is either deleted or replaced by the reactive site of an antithrombin active serine protease inhibitor cleavable with thrombin.

2. An $\alpha_2$-PI derivative as claimed in claim 1, wherein the flanking regions of the said reactive site(s) are also deleted or replaced.

3. An $\alpha_2$-PI derivative as claimed in claim 1 or 2, wherein the amino terminal end is Asn. Gln. Glu. Gln. Val. Ser. Pro. Leu. Thr. Leu.

4. An $\alpha_2$-PI derivative as claimed in claim 1, 2 or 3, wherein the carboxyl terminal end is Leu. Lys. Leu. Val. Pro. Pro. Met. Glu. Glu. Asp. Tyr. Pro. Gln. Phe. Gly. Ser. Pro. Lys.

5. DNA coding for the protein of claim 1.

6. A plasmid containing the DNA according to claim 5.

7. A procaryotic or eucaryotic cell transformed with a DNA containing the DNA claimed in claim 5.

8. A procaryotic or eucaryotic cell transformed with a plasmid according to claim 6.

9. A process for preparing the protein of claim 1, 2, 3 or 4, which comprises culturing the procaryotic or eucaryotic cells according to claim 7 or 8.

Claims for the following Contracting State : GR

1. A process for the preparation of an $\alpha_2$-plasmin inhibitor ($\alpha_2$-PI) derivative having essentially the amino acid sequence of $\alpha_2$-PI but for the reactive site cleavable with plasmin, which reactive site is either deleted or replaced by the reactive site of an antithrombin active serine protease inhibitor cleavable with thrombin, characterized by expressing a gene coding for this $\alpha_2$-PI derivative in a host cell.

2. A process as claimed in claim 1, characterized in that the flanking regions of the said reactive site(s) are also deleted or replaced.

3. A process as claimed in claim 1 or 2, characterized in that the amino terminal end is Asn. Gln. Glu. Gln. Val. Ser. Pro. Leu. Thr. Leu.

4. A process as claimed in claim 1, 2 or 3, characterized in that the carboxyl terminal end is Leu. Lys. Leu. Val. Pro. Pro. Met. Glu. Glu. Asp. Tyr. Pro. Gln. Phe. Gly. Ser. Pro. Lys.

5. DNA coding for the protein as defined in claim 1.

6. A plasmid containing the DNA according to claim 5.

7. A procaryotic or eucaryotic cell transformed with a DNA containing the DNA claimed in claim 5.

8. A procaryotic or eucaryotic cell transformed with a plasmid according to claim 6.


Claims for the following Contracting State : ES

1. A process for the preparation of an $\alpha_2$-plasmin inhibitor ($\alpha_2$-PI) derivative having essentially the amino acid sequence of $\alpha_2$-PI but for the reactive site cleavable with plasmin, which reactive site is either deleted or replaced by the reactive site of an antithrombin active serine protease inhibitor cleavable with thrombin, characterized by expressing a gene coding for this $\alpha_2$-PI derivative in a host cell.

2. A process as claimed in claim 1, characterized in that the flanking regions of the said reactive site(s) are also deleted or replaced.

3. A process as claimed in claim 1 or 2, characterized in that the amino terminal end is Asn. Gln. Glu. Gln. Val. Ser. Pro. Leu. Thr. Leu.

4. A process as claimed in claim 1, 2 or 3, characterized in that the carboxyl terminal end is Leu. Lys. Leu. Val. Pro. Pro. Met. Glu. Glu. Asp. Tyr. Pro. Gln. Phe. Gly. Ser. Pro. Lys.

# FIG.1

$\alpha_2$-plasmin inhibitor-coding region

SphI
PstI
AccI
(EcoRI)
NcoI
PstI
SmaI
FokI
FokI
PstI
EcoRI
FokI
PstI
SacI
SacI
SmaI
HindIII
NcoI
(FokI)
XbaI
BamHI
EcoRI

lacZ'
(Polylinker)

HindIII

SmaI

Kanamycin-resistant gene

ClaI

pαAP8

ori

# FIG.2

Hae II
ori
HaeII
NdeI
SnaI
PvuII
HpaI
trp Promoter
pHSG 741
rrnB Terminator
Hae II
Ampicillin-resistant gene
MstI
PvuI

Polylinker

NcoI HpaI PvuII AsuII SacI BalI XhoI SphI PstI SalI XbaI BamHI SmaI KpnI SacI EcoRI Eco47III MstI HindIII

FIG. 3

pαAP 213

Hae II

HaeII
NdeI
SnaI
ori

Mst I
PvaI

Ampicillin-
resistant gene

PvuII
trp
Promoter

α₂-plasmin inhibitor-
coding region

rrnB
Terminator

HpaI
(NcoI)
PstI
(+236)
HaeII
PstI
EcoRI
(+676)
PstI

Hae II
XbaI